Europäisches Patentamt

European Patent Office

Office européen des brevets

(19)

(11) Publication number : **0 312 515 B1**

# (12) EUROPEAN PATENT SPECIFICATION

(45) Date of publication of patent specification :
06.05.92 Bulletin 92/19

(51) Int. Cl.⁵ : **A61F 5/01**

(21) Application number : **88850328.1**

(22) Date of filing : **04.10.88**

(54) Orthosis.

(30) Priority : **15.10.87 SE 8702813**

(43) Date of publication of application :
**19.04.89 Bulletin 89/16**

(45) Publication of the grant of the patent :
**06.05.92 Bulletin 92/19**

(84) Designated Contracting States :
**CH DE ES GB IT LI NL**

(56) References cited :
**DE-A- 3 440 521**
**US-A- 4 320 747**

(73) Proprietor : **PI MEDICAL AB**
**P O Box 67**
**S-751 03 Uppsala (SE)**

(72) Inventor : **Carlsson, Roger**
**Källbogatan 14**
**S-212 33 Malmö (SE)**
Inventor : **Edevret, Roland**
**Smabruksvägen 4**
**S-291 69 Kristianstand (SE)**

(74) Representative : **Barnieske, Hans Wolfgang et al**
**c/o H.W. Barnieske Patentbyrä AB S:ta Ragnhildsgatan 24-26 P.O. Box 25**
**S-151 21 Södertälje 1 (SE)**

EP 0 312 515 B1

## Description

The present invention relates to an orthosis for stabilizing the joint of an extremity, preferably a knee-joint, by limiting its movement, consisting of a relatively rigid upper part and a relatively rigid lower part, designed to be secured above and below the extremity joint, respectively, and at least one pivot joint consisting of an upper joint part joined to the upper part of the orthosis and a lower joint part joined to the lower part of the orthosis, the joint parts being provided with stop means co-operating with each other to provide two stop positions determining the minimum and maximum bending angle, respectively, of the orthosis.

It is well known that orthoses constitute a valuable aid in the conservative or post-operative treatment of pain or other problems occurring in joints of the extremities. People performing sports frequently use such orthoses since their joints are subjected to particularly great strain. However, they are also used for other types of joint problems. Since the knee-joint, the joint in the human body which is subjected to the greatest load, has the highest incidence of problems of all the joints in the body, it is natural that knee-joint orthoses should be the most usual type.

An extremely usual type of orthosis consists of a sleeve of elastic material which is secured around the thigh and around the lower leg. The sleeve is generally provided with an opening for the patella. Such orthoses have proved valuable in treating many different types of problems, but nevertheless have a number of drawbacks. It is, for instance, difficult to achieve absolute immobility and stability in certain directions while still retaining freedom of movement in other directions, and only an extremely limited stability against rotation can be achieved with this type of orthosis. Another problem is the formation of folds behind the knee when the leg is bent. Yet another drawback is that this type of orthosis is not suitable for use in water, for instance when bathing.

Existing rigid orthoses as for example shown in US 4 320 747 comprises an upper and an lower part joined together by a pivot joint are not satisfactory either since they are relatively complicated and thus expensive in manufacture. Special limiting devices, e.g. pins applied in holes in one part of the pivot joint, have been used to achieve the desired limitation in turning angle of a pivot joint. This limits the setting possibilities for the bending angle of the pivot joint to the positions determined by the pin-holes and no continuous adjustment between these positions can therefore be achieved.

It is an object of the invention to provide an orthosis which eliminates the above-mentioned drawbacks and which has extremely satisfactory lateral and rotary stability as well as permitting optional adjustment of the angles of movement of the orthosis.

Furthermore, there is nothing to prevent the orthosis being used while bathing.

Other objects, features and advantages of the present invention will be become appear from the subsequent description and the appended claims, taken in conjunction with the accompanying drawings in which,

Figure 1 shows in perspective an orthosis according to the invention,

Figure 2 shows from the side a first pivot joint part in the orthosis according to Figure 1, and

Figure 3 shows from the side a second pivot joint part complementary to the first part.

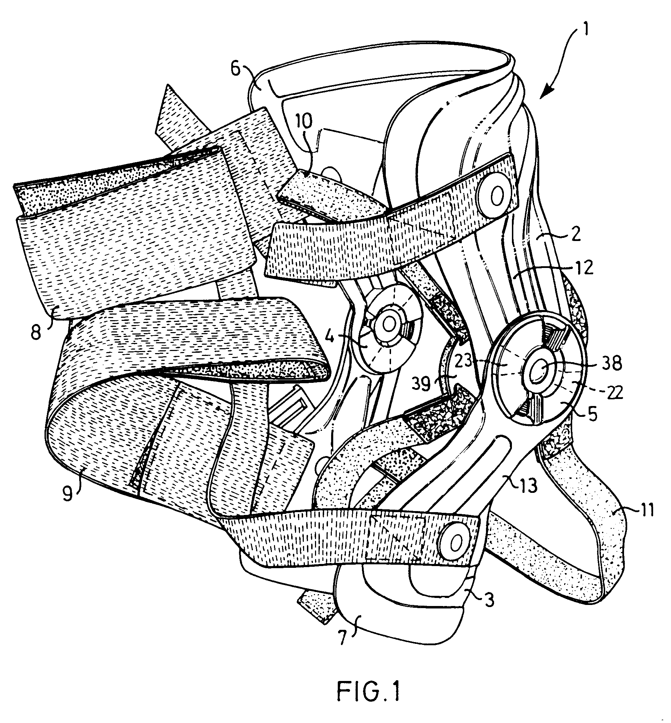

Referring to Fig 1, the orthosis 1 which is designed for a knee-joint, consists of an upper part 2 to be secured around the thigh, and a lower part 3 to be secured around the lower leg. The two parts 2, 3, which are made of a relatively rigid plastic such as polypropene or polythene, are flexibly joined together by means of two pivot joints arranged one on each side of the knee. Each orthosis part 2, 3 comprises a surrounding part 6 and 7, respectively, which when in position on the leg is designed to surround most of the relevant extremity and is secured around the extremity by means of Velcro strips 8 and 9, respectively. Straps 10 and 11, respectively, secured in a condyle plate 39, are used to keep the orthosis in the desired position with respect to the knee. Tapering uprights 12, 13 connect the surrounding parts 6 and 7, respectively, of the orthosis parts 2, 3, to the parts 4 and 5, respectively of the pivot joint. The pivot joint parts 4, 5 are made in one piece with the relevant orthosis part 2, 3. The orthosis parts 2, 3 and joint parts 4, 5 are made of plastic and are suitably compression moulded or injection moulded.

The orthosis in Fig 1 comprises two pivot joints located one on each side of the knee-joint and coinciding with the joint axis. Since both pivot joints are the same, only one of them will be described in the following.

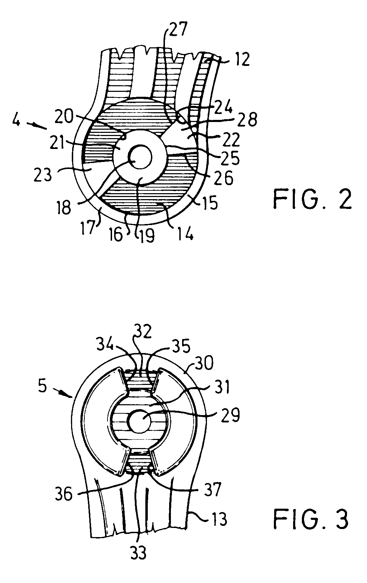

Referring to Fig 2, the pivot joint part 4 of the upper orthosis part 2 consists of a substantially circular plate 14, constrained by an annular flange 15 forming an inner cylinder surface 16, the cylinder axis coinciding substantially with the joint axis. The edge surface 17 of the flange 15 running perpendicular to the pivot axis is smooth and forms an axial journalling surface. In the centre of the plate 14 is a through-hole 18 for a shaft pin. The hole 18 stretches through a central, cylindrical protrusion 19 with outer cylinder surface 20. The cylinder axis thus substantially coincides with the joint axis. The end surface 21 of the protrusion 19 is flat and may be designed as a journalling surface. Two shoulders 22, 23 located diametrically opposite each other protrude out over the annular flange 15 and the protrusion 19. Each shoulder 22, 23 has an outer arc-shaped cylinder surface 24 and an inner arc-shaped cylinder surface 25. The axes of the cylinder surfaces 24 and 25 substantially coincide with the axis of the pivot joint. The lateral surfaces 26 and 27 of the shoulders 22, 23 are flat and are located in a radial plane through the joint axis. The end surface 28 may be designed as a journalling surface.

The lower pivot joint part 5, joined to the lower orthosis part 3, is designed complementarily to the upper pivot joint part 4 and has a central through-hole for application opposite the hole 18. Exactly like the upper pivot joint part 4, this part is provided with an arc-shaped flange 30, the annular end surface of which cooperates with the end surface 17 of the flange 15, forming an axial bearing when fitted together. The inner cylinder surface of the flange 30 cooperates with the outer cylinder surfaces 24 of the shoulders 22, 23 of the upper pivot joint part to form a radial bearing. The lower pivot joint part 5 also includes a cylindrical protrusion 31. The outer cylinder surface of this protrusion cooperates with the inner cylinder surfaces 25 of the upper shoulders 22, 23 to form another radial bearing. The lower pivot joint part 5 also includes a pair of diametrically located shoulders 32, 33 with outer and inner cylindrical limiting surfaces and lateral surfaces 34, 35 and 36, 37, respectively, which are located in a radial plane running through the joint axis. The lateral surfaces 34, 35 and 36, 37, respectively, on the shoulders 32, 33 form stop means which cooperate with the lateral surfaces 26, 27 of the shoulders 22, 23 in the other part of the pivot joint. The minimum and maximum range of joint movement of the orthosis is thus determined by the contact between the lateral surfaces.

An insert of suitable thickness, placed in the arc-shaped, channel-like grooves formed between two opposing lateral surfaces and the cylinder surfaces on an annular flange and a central protrusion enables the joint angle and its position to be set individually as required by a particular patient.

When a suitable bending angle has been achieved by means of the insert, the joint part 4 is fitted over the part 5 so that the shoulders 22, 23 and 32, 33 fit into the proper grooves. The shaft pin 38 is then passed through the holes 18, 29 and secured in order to hold the parts 4 and 5 of the pivot joint together in relation to each other.

A soft, cap-shaped support 39, a condyle plate, may be secured to one side of the orthosis in the joint area as shown in Fig 1, to provide lateral support for the knee and to position the orthosis in relation to the knee.

The orthosis 1 described above offers a medical aid which limits movement and provides excellent stabilization of the knee-joint both laterally and in rotary direction, as well as giving the knee extremely good varus-valgus stability. Despite the high rigidity of the orthosis it weighs little and its shape is easily accepted by the user.

Naturally, the main area of application for orthoses is knee-joints, but this does not prevent the principle from being used for other extremity joints.

## Claims

1. An orthosis for stabilizing the joint of an extremity, preferably a knee-joint, by limiting its movement, consisting of a relatively rigid upper part (2) and a relatively rigid lower part (3), designed to be secured above and below the joint, respectively, at least one pivot joint consisting of an upper joint part (4) joined to the upper part (2) of the orthosis and a lower joint part (5) joined to the lower part (3) of the orthosis, the joint parts being provided with stop means (22, 23, 32, 33) cooperating with each other to provide two stop positions determining the minimum and maximum bending angle, respectively, each pivot joint part (4, 5) with its stop means (22, 23 and 32, 33, respectively) is made in one piece with the part (2 or 3) of the orthosis pertaining theretor, **charaterized** to that each joint part (4, 5) is provided with an arc-shaped flange (15, 30) having an end surface (17) running perpendicular to the joint axis, the end surfaces cooperating with each other to form an axial journal.

2. An orthosis as claimed in claim 1, one joint part being provided with a shoulder (22, 23, 32, 33), **characterized** in that the arc-shaped flange portion (15, 30) of the other joint part comprises an inner cylinder section (16), the cylinder axis substantially coinciding with the joint axis, and wherein the shoulder comprises an outer cylinder portion (28) corresponding to the cylinder section, to form a first radial journalling position.

3. An orthosis as claimed in claim 1 or 2, one of the joint parts being provided with a shoulder (22, 23, 32, 33), **characterized** in that the other joint part comprises a cylindrical protrusion (19, 31) located centrally about the joint axis and having an outer cylinder section (20), and wherein the shoulder (22, 23, 32, 33) comprises an inner cylinder portion (17) corresponding to the outer cylinder section to form a second radial journalling position.

4. An orthosis as claimed in claims 1 - 3, **characterized** in that the orthosis parts (2, 3) and joint parts (4, 5) are made of plastic.

5. An orthosis as claimed in claims 1 - 4, for a knee-joint, **characterized** in a soft, cup-shaped support plate (39) arranged to rest against one side of the knee.

## Patentansprüche

1. Orthese zur Stabilisierung des Gelenkes eines Gliedes, vorzugsweise eines Kniegelenkes, durch Beschränkung seiner Bewegung, bestehend aus einem verhältnismässig steifen oberen Teil (2) und einem verhältnismässig steifen unteren Teil (3), die dazu ausgebildet sind, über bzw. unter dem Gelenk befestigt zu sein, mindestens einem Drehgelenk, das aus einem mit dem oberen Teil (2) der Orthese verbundenen oberen Gelenkteil (4) und einem mit dem unteren Teil (3) der Orthese verbundenen unteren Gelenkteil (5) besteht, wobei die Gelenkteile mit Begrenzungsmitteln (22,23,32,33) versehen sind, die zusammenwirken, um zwei den minimalen bzw. maximalen Beugewinkel bestimmenden Begrenzungslagen zu ergeben, wobei jeder Drehgelenkteil (4,5) mit seinen Begrenzungsmitteln (22,23 bzw. 32,33) einteilig mit dem ihm zugehörigen Teil (2 oder 3) der Orthese ausgebildet ist, dadurch gekennzeichnet, dass jeder Gelenkteil (4,5) mit einem bogenförmigen Flansch (15,30) versehen ist, der eine rechtwinklig zur Gelenkachse verlaufende Endfläche (17) aufweist, wobei die Endflächen miteinander zusammenwirken, um ein axiales Lager zu bilden.

2. Orthese nach Anspruch 1, wobei ein Gelenkteil mit einer Schulter (22,23,32,33) versehen ist, dadurch gekennzeichnet, dass der bogenförmigen Flanschteil (15,30) des anderen Gelenkteils einen inneren zylindrischen Abschnitt (16) umfasst, wobei die Zylinderachse im wesentlichen mit der Gelenkachse übereinstimmt, und wobei die Schulter einen dem inneren zylindrischen Abschnitt entsprechenden äusseren zylindrischen Teil (28) umfasst, um eine erste radiale Lagerposition zu bilden.

3. Orthese nach Anspruch 1 oder 2, wobei einer der Gelenkteile mit einer Schulter (22,23,32,33) versehen ist, dadurch gekennzeichnet, dass der andere Gelenkteil einen zylindrischen Vorsprung (19,31) umfasst, der um die Gelenkachse zentriert angeordnet ist und einen äusseren zylindrischen Abschnitt (20) aufweist, und wobei die Schulter (22,23,32,33) einen dem äusseren zylindrischen Abschnitt entsprechenden inneren zylindrischen Teil (17) umfasst, um eine zweite radiale Lagerposition zu bilden.

4. Orthese nach den Ansprüchen 1 bis 3, dadurch gekennzeichnet, dass die Orthesenteile (2,3) und die Gelenkteile (4,5) aus Kunststoff gefertigt sind.

5. Orthese nach den Ansprüchen 1 bis 4 für ein Kniegelenk, gekennzeichnet durch eine weiche schalenförmige Tragplatte (39), die so angeordnet ist, dass sie auf einer Seite des Knies ruht.

**Revendications**

1. Orthèse pour stabiliser l'articulation d'une extrémité, de préférence d'une articulation de genou, en limitant son mouvement, consistant en une partie supérieure (2) relativement rigide et une partie inférieure (3) relativement rigide destinées à être fixées respectivement au-dessus et au-dessous de l'articulation, au moins une articulation pivotante consistant en une partie supérieure d'articulation (4) reliée à la partie supérieure (2) de l'orthèse et une partie inférieure d'articulation (5) reliée à la partie inférieure (3) de l'orthèse, les parties d'articulation étant pourvues de moyens d'arrêt (22,23,32,33) qui coopèrent entre eux pour fournir deux positions d'arrêt qui déterminent respectivement l'angle de pliure minimum et maximum, chaque partie d'articulation pivotante (4,5) et ses moyens d'arrêt (22,23 respectivement 32,33) étant d'une seule pièce avec la partie (2 ou 3) de l'orthèse qui lui correspond, caractérisée en ce que chaque partie d'articulation (4,5) est pourvue d'une bride (15,30) en forme d'arc qui présente une surface terminale (17) qui s'étend perpendiculairement à l'axe de l'articulation, les surfaces terminales coopérant entre elles pour former un palier axial.

2. Orthèse selon la revendication 1, une partie d'articulation étant pourvue d'un épaulement (22,23,32,33), caractérisée en ce que la partie de bride (15,30) en forme d'arc de l'autre partie d'articulation comporte une section de cylindre intérieure (16), l'axe du cylindre coïncidant essentiellement avec l'axe de l'articulation, et dans laquelle l'épaulement comporte une partie de cylindre extérieure (28) qui correspond à la section de cylindre intérieure afin de former une première position de palier radial.

3. Orthèse selon la revendication 1 ou 2, une des parties d'articulation étant pourvue d'un épaulement (22,23,32,33), caractérisée en ce que l'autre partie d'articulation comporte une saillie cylindrique (19,31) située de façon centrée autour de l'axe de l'articulation et qui comporte une section de cylindre extérieure (20), et dans laquelle l'épaulement (22,23,32,33) comporte une partie de cylindre intérieure (17) qui correspond à la section de cylindre extérieure afin de former une deuxième position de palier radial.

4. Orthèse selon les revendications 1 à 3, caractérisée en ce que les parties (2,3) d'orthèse et les parties d'articulation (4,5) sont en matière plastique.

5. Orthèse selon les revendications 1 à 4 pour une articulation de genou, caractérisée par une plaque de support (39) molle en forme de coupe disposée de façon à reposer contre l'un des côtés du genou.

FIG.1

FIG. 2

FIG. 3